(19) Europäisches Patentamt
European Patent Office
Office européen des brevets

(11) Publication number: **0 285 287**
**A2**

# (12) EUROPEAN PATENT APPLICATION

(21) Application number: 88302319.4

(51) Int. Cl.⁴: **A61K 31/55**

(22) Date of filing: 17.03.88

Claims for the following Contracting States: ES + GR.

(30) Priority: 23.03.87 US 29289

(43) Date of publication of application: 05.10.88 Bulletin 88/40

(84) Designated Contracting States: AT BE CH DE ES FR GB GR IT LI LU NL SE

(71) Applicant: **SMITHKLINE BECKMAN CORPORATION**
**One Franklin Plaza P O Box 7929**
**Philadelphia Pennsylvania 19103(US)**

(72) Inventor: **Bondinell, William Edward**
**1512 Franklin Lane**
**Wayne Pennsylvania 19087(US)**
Inventor: **Ormsbee III, Herbert Stowell**
**652 Pugh Road**
**Wayne Pennsylvania 19087(US)**

(74) Representative: **Waters, David Martin, Dr. et al**
**Smith Kline & French Laboratories Ltd.**
**Patent Department Mundells**
**Welwyn Garden City Hertfordshire AL7 1EY(GB)**

## (54) 3-Benzazepine compounds for use in treating gastrointestinal motility disorders.

(57) The use of a compound of the formula (I) :

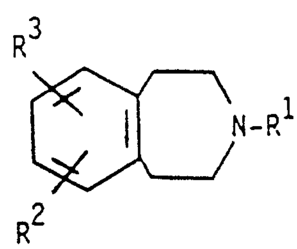

(I)

or a pharmaceutically acceptable acid addition salt thereof, in the manufacture of a medicament for the treatment of gastrointestinal motility disorders is described wherein $R^1$ is hydrogen, $C_1$-$C_6$ alkyl or $C_3$-$C_5$ alkenyl; $R^2$ is hydrogen, halogen, $CF_3$, $C_1$-$C_6$ alkyl or $R^4O$; $R^3$ is halogen, $CF_3$, $C_1$-$C_6$ alkyl, hydroxy, $C_1$-$C_6$ alkoxy,. S-($C_1$-$C_6$) alkyl, S-trifluoromethyl, $NO_2$, $SO_n$-phenyl where the phenyl is optionally substituted by hydroxy, $C_1$-$C_6$ alkoxy, halogen, $CF_3$, $C_1$-$C_6$ alkyl, $SO_n$($C_1$-$C_6$) alkyl, $SO_nCF_3$, $SO_n$-phenyl or $SO_2NR^5R^6$; $R^4$ is hydrogen, $C_1$-$C_6$ alkyl, or $C_1$-$C_6$alkanoyl; $R^5$ and $R^6$ are hydrogen or $C_1$-$C_6$ alkyl; and n is 0, 1 or 2; provided $R^2$ is not hydrogen when $R^3$ is S-phenyl in the 6-position and provided that when $R^2$ is hydroxy in the 8-position and $R^1$ is $C_1$-$C_6$ alkyl, $R^3$ may be halogen or $CF_3$ only if it is in the 6-or 9-position.

EP 0 285 287 A2

# 3-BENZAZEPINE COMPOUNDS FOR USE IN TREATING GASTROINTESTINAL MOTILITY DISORDERS

This invention relates to 3-benzazepine compounds for use in treating gastrointestinal motility disorders, pharmaceutical compositions containing them and their use in the manufacture of medicaments for treating gastrointestinal motility disorders.

It has now been found that the 3-benzazepine compounds are useful therapeutically for gastroesophageal reflux disease and disorders of delayed gastric emptying of various etiologies including diabetes, surgery, and idiopathic delayed emptying. They may also be useful in treating disorders of upper GI motility, aspiration, early satiety, anorexia nervosa, and in diagnostic radiology or to facilitate intubation.

These compounds are known from US Patent Nos. 4,265,890, 3,671,519, 3,483,185, 4,469,634 and GB Patent No. 1,268,243 and have been reported as antipsychotics, anti-emetics, analgesics, hypoglycemic agents, $\alpha_2$-antagonists and narcotic antagonists.

According to the present invention there is provided the use of a compound of the formula (I):

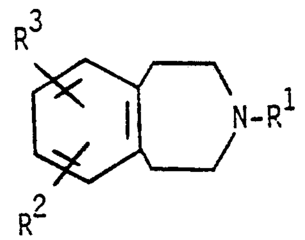

(I)

in which:

$R^1$ is hydrogen, $C_1$-$C_6$ alkyl or $C_3$-$C_5$ alkenyl;

$R^2$ is hydrogen, halogen, $CF_3$, $C_1$-$C_6$ alkyl or $R^4O$;

$R^3$ is halogen, $CF_3$, $C_1$-$C_6$ alkyl, hydroxy, $C_1$-$C_6$ alkoxy, S-($C_1$-$C_6$ alkyl), S-trifluoromethyl, $NO_2$, $SO_n$-phenyl where phenyl is optionally substituted by hydroxy, $C_1$-$C_6$ alkoxy, halogen, $CF_3$, $C_1$-$C_6$ alkyl, $SO_n$($C_1$-$C_6$) alkyl, $SO_nCF_3$, $SO_n$phenyl or $SO_2NR^5R^6$;

$R^4$ is hydrogen, $C_1$-$C_6$ alkyl, or $C_1$-$C_6$ alkanoyl;

$R^5$ and $R^6$ are hydrogen or $C_1$-$C_6$ alkyl; and n is 0, 1, or 2;

provided $R^2$ is not hydrogen when $R^3$ is S-phenyl in the 6-position and provided that when $R^2$ is hydroxy in the 8-position and $R^1$ is $C_1$-$C_6$ alkyl, $R^3$ may be halogen or $CF_3$ only if it is in the 6-or 9 position;

or a pharmaceutically acceptable acid addition salt thereof, in the manufacture of a medicament for the treatment of gastrointestinal motility disorders.

A group of compounds of formula (I) is that in which $R^2$ is hydrogen, hydroxy or methoxy, $R^3$ is $SO_n$-phenyl or S-($C_1$-$C_6$ alkyl), and $R^1$ is hydrogen or methyl.

Particular compounds of formula (I) are those in which $R^3$ is in the 7-position and $R^2$ is in the 8-position; or $R^3$ is in the 6-position and $R^2$ is in the 7-position.

Specific compounds of formula (I) are:

8-hydroxy-3-methyl-7-phenylthio-2,3,4,5-tetrahydro-1H-3-benzazepine

7-hydroxy-3-methyl-6-phenylthio-2,3,4,5-tetrahydro-1H-3-benzazepine

7-hydroxy-2,3,4,5-tetrahydro-1H-3-benzazepine

3-methyl-7-phenylthio-2,3,4,5-tetrahydro-1H-3-benzazepine

The compounds of formula (I) are known to the art and may be prepared as follows:

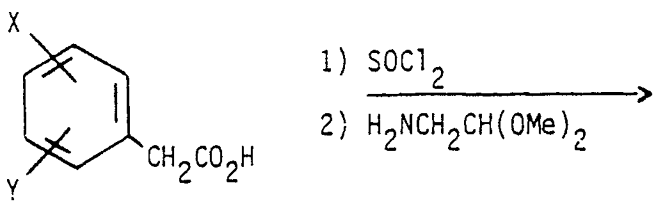


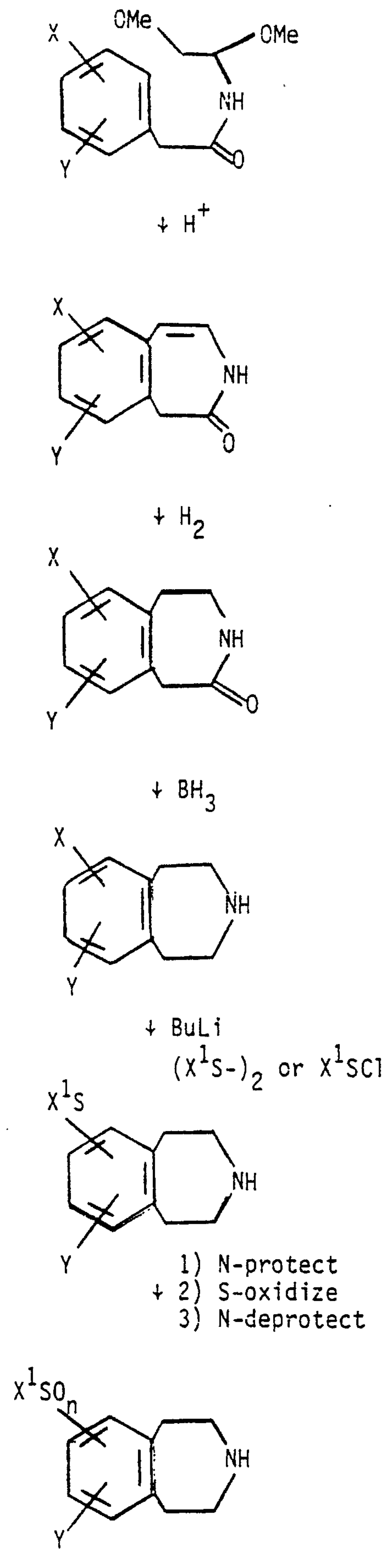


X = hydrogen, halogen, alkoxy, alkyl, $CF_3$

Y = hydrogen, halogen, alkoxy, alkyl, $CF_3$, $NO_2$

$X^1$ = $C_1$-$C_6$ alkyl, $CF_3$, phenyl

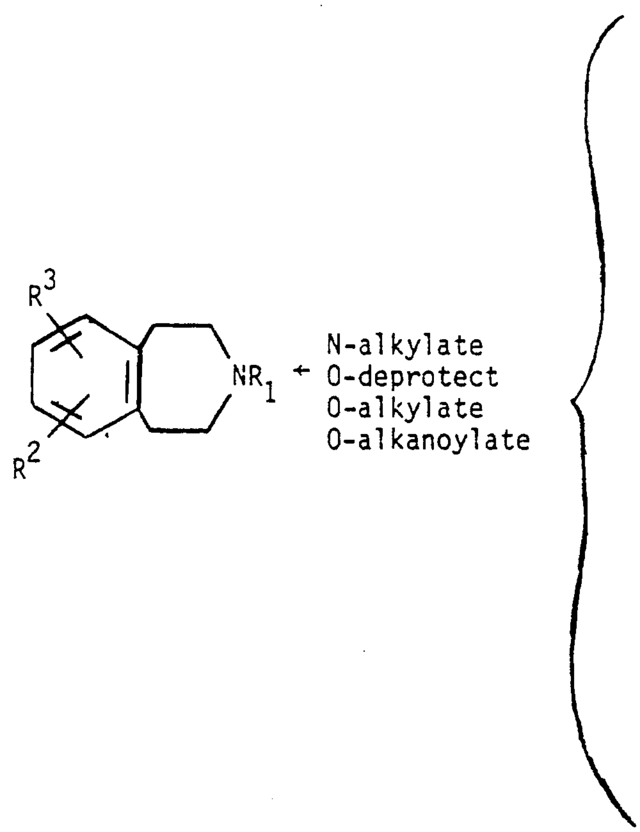

According to the above procedure, a substituted phenylacetic acid is first reacted with thionyl chloride, then aminoacetaldehyde dimethylacetal to give the corresponding N-(2,2-dimethoxyethyl)-benzeneacetamide. Acid catalyzed cyclization followed by hydrogenation and reduction with borane afford the corresponding substituted 2,3,4,5-tetrahydro-1H-3-benzazepine.

The substituted 2,3,4,5-tetrahydro-1H-3-benzazepine serves as a general intermediate which may be N-alkylated, 0-deprotected, or 0-alkanoylated to give the halo, alkyl, hydroxy, alkoxy or alkanoyloxy substituted benzazepine; or it may be treated with N-butyllithium and a disulfide $(X'S-)_2$ or sulfenyl chloride (X'-SCl) to give the thio-substituted compounds or when Y = $NO_2$ may be prepared by nitration. These in turn may undergo further modification by N-alkylation, O-deprotection, O-alkylation or O-alkanoylation to give the desired product.

This thio-substituted benzazepine may alternatively be N-protected, S-oxidized and N-deprotected to give the sulfonyl and sulfinyl substituted benzazepines. They may undergo further modification by N-alkylation, O-deprotection, O-alkylation or O-alkanoylation to give the desired compounds. All procedures are carried out by standard methods known to those skilled in the art and may be carried out in various order of steps to prepare the desired compounds.

The compounds of formula (I) form pharmaceutically acceptable acid addition salts with organic or inorganic acids. Examples of these acids are hydrochloric, hydrobromic, sulfuric, phosphoric, acetic, tartaric, citric, maleic, lactic, oxalic, succinic, methanesulfonic, and benzenesulfonic acids. The salts are formed according to methods known to the art. If the product is isolated as an acid addition salt, it may be treated with an inorganic or organic base, such as aqueous sodium hydroxide, sodium carbonate, triethylamine, etc., and converted to the corresponding free base. The base can then be treated with an appropriate acid, for example, in an aqueous miscible solvent, such as a lower alcohol preferably methanol or ethanol, to give the desired salt.

The effect of the pharmacologically active compounds of formula (I) on gastrointestinal motility is demonstrated in test procedures as follows:

(1) an increase in resting pressure of the lower esophageal sphincter (LES) in dogs; and

(2) an increase in the rate of gastric emptying in rats.

Methods for Determination of LES Pressure in the Anesthetized Dog

Mongrel or beagle dogs, male and female, are anesthetized using sodium pentobarbital (35.0 mg/kg., i.v.). Sodium pentobarbital is then continuously infused (approximately 6.0 mg/kg/hr) to maintain deep anesthesia. Blood pressure is monitored via a catheter surgically implanted into the femoral artery and attached to a Gould-Statham P23ID transducer. A catheter is also implanted into the femoral vein to administer test drugs. Respiration is maintained by an endotracheal tube attached to a respirator. A continuously perfused manometric catheter system including a Dent sleeve to measure sphincter pressure (Dent, Gastroenterology 71:263-267, 1976) is inserted into the esophagus and positioned so that intraluminal pressure is recorded from the body of the esophagus, the lower esophageal sphincter (LES) and the fundus of the stomach. The Dent sleeve catheter is perfused at a rate of 0.5 ml of water per minute for each lumen of the catheter by using an Arndorfer Hydraulic Capillary Infusion System. A cannula is implanted into the gastric antrum to allow drainage of the perfusate solution and prevent intestinal distension. Continuous tracings of esophageal, LES and fundus pressure are monitored on a Grass Polygraph (Model 7D). Correct positioning of the Dent sleeve is verified by noting a high pressure zone at the LES and by administration of an intravenous dose of 5-HT (usually 10-15 mcg/kg) which contracts the LES while having little or no recordable effect on either the body of the esophagus or the fundus. After verification of placement of the sleeve, the animal is allowed to stabilize for approximately 30 minutes.

Compounds are administered intravenously or intraduodenally. In most cases, succeeding doses of the same or different compounds are given only after the LES pressure has returned to approximate pre-dosing baseline values. In all cases, the magnitude of change in LES pressure is determined from the baseline pressure immediately prior to each treatment to the maximum pressure during treatment. Since the compounds used usually produce an immediate effect on LES pressure, no pretreatment time prior to measurement is necessary during this testing.

Direct assay techniques are used to estimate an Effective Dose 20 ($ED_{20}$) for the test compound in

individual animals. The mean $ED_{20}$ and 95% confidence limits are determined using the individual $ED_{20}$ values from a group of animals (N = ≥3) that received the same treatment. The $ED_{20}$ is the dose which increases LES pressure 20 mm Hg.

Table I gives the $ED_{20}$ for certain compounds of this invention.

Table I

| Compound | | $ED_{20}$ in mcg/kg i.v. | $ED_{20}$ in mcg/kg i.d. |
|---|---|---|---|
| 1. | 7-hydroxy-3-methyl-6-phenylthio-2,3 4,5-tetrahydro-1H-3-benzazepine | 63 | 263 |
| 2. | 8-hydroxy-3-methyl-7-phenylthio-2,3 4,5-tetrahydro-1H-3-benzazepine | 14 | ~1000 |
| 3. | 3-methyl-7-methylthio-2,3,4,5-tetrahydro-1H-3-benzazepine | 273 | 236 |
| 4. | 7-hydroxy-2,3,4,5-tetrahydro-1H-3-benzazepine | 348 | - |
| 5. | 3-methyl-7-nitro-8-phenylthio-2,3,4,5-tetrahydro-1H-3-benzazepine | 1200 | - |

Gastric Emptying in the Rat

Fasted rats are administered 0.5 µCi of $Na_2^{51}CrO_4$ (0.2 ml vol) into the stomach with an oral feeding tube. Compounds for evaluation or vehicle controls are administered either 15 minutes before (oral administration) or simultaneously with (intravenous administration) the test meal. After 35 minutes the rats are killed by cervical dislocation and the stomach is removed. Gastric emptying is measured from the amount of $^{51}Cr$ remaining in the stomach at death. The rate of gastric emptying as compared to control is determined.

The method of treating gastrointestinal motility disorders comprises administering internally to a subject in need of said treatment an effective amount of a compound of formula (I) or a pharmaceutically acceptable acid addition salt thereof. The compound will preferably be administered in a dosage unit form orally or parenterally. Advantageously, equal doses will be administered one to four times daily with the daily dosage regimen being about 1 mg. to about 1000 mg., preferably from 2 mg. to 400 mg. The method described above is useful for treating gastrointestinal motility disorders.

Preferably, the compounds of formula (I) are administered in conventional dosage unit forms prepared by combining an appropriate dose of the compound with standard pharmaceutical carriers. The dosage units will contain the active ingredient in an effective amount selected from about 0.25 mg. to about 250 mg., preferably 0.5 mg. to 100 mg.

The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the like. Similarly, the carrier or diluent can include any time delay material well-known to the art, such as glyceryl monostearate

or glyceryl distearate alone or with a wax.

A wide variety of pharmaceutical forms can be employed. Thus, if a solid carrier is used, the preparation can be tableted, placed in a hard gelatin capsule in powder or pellet form or in the form of a trouche or lozenge. The amount of solid carrier will vary widely but preferably will be from about 25 mg. to about 1 g. If a liquid carrier is used, the preparation will be in the form of a syrup, emulsion, soft gelatin capsule, sterile injectable liquid such as an ampoule or an aqueous or nonaqueous liquid suspension.

The pharmaceutical compositions are prepared by conventional techniques involving procedures such as mixing, granulating and compressing when necessary or variously mixing and dissolving the ingredients as appropriate to the desired composition.

One skilled in the art will recognize that in determining the amounts of the compound needed to produce the desired pharmacological effect without toxic side effects, the activity of the particular compound as well as the size of the host animal must be considered.

This invention also includes pharmaceutical compositions comprising a pharmaceutically acceptable carrier and a compound of the following formula (II):

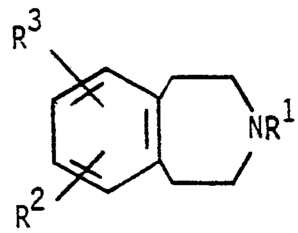

(II)

in which:

$R^1$ is hydrogen, $C_1$-$C_6$ alkyl or $C_3$-$C_5$ alkenyl;

$R^2$ is hydrogen, halogen, $CF_3$, $C_1$-$C_6$ alkyl or $R^4O$;

$R^3$ is halogen, $CF_3$, $NO_2$, S-($C_1$-$C_6$) alkyl, S-trifluoromethyl, $SO_n$-phenyl where phenyl is optionally substituted by hydroxy, $C_1$-$C_6$ alkoxy, halogen, $CF_3$, $C_1$-$C_6$ alkyl, $SO_n$($C_1$-$C_6$) alkyl, $SO_nCF_3$, $SO_n$phenyl or $SO_2NR^5R^6$;

$R^4$ is hydrogen, $C_1$-$C_6$ alkyl or $C_1$-$C_6$ alkanoyl;

$R^5$ and $R^6$ are hydrogen or $C_1$-$C_6$ alkyl; and

n is 1, or 2, or if $R^3$ is in the 7-or 8-position n may be 0;

provided that when $R^2$ is hydroxy in the 8-position and $R^1$ is $C_1$-$C_6$ alkyl, $R^3$ may be halogen or $CF_3$ only if it is in the 6-or 9-position; or a pharmaceutically acceptable acid addition salt thereof.

Such compositions are particularly useful in the treatment of gastrointestinal motility disorders.

Specific compounds for use in this invention are:

3-methyl-7-phenylthio-2,3,4,5-tetrahydro-1H-3-benzazepine or

8-hydroxy-3-methyl-7-phenylthio-2,3,4,5-tetrahydro-1H-3-benzazepine,

or a pharmaceutically acceptable acid addition salt thereof.

The following examples illustrate the invention but are not to be construed as limiting the scope thereof.

## EXAMPLE 1

### 7-Hydroxy-2,3,4,5-tetrahydro-1H-3-benzazepine

A mixture of 3-methoxyphenylacetic acid (47.7 g, 0.287 mol), thionyl chloride (50 mL) and N,N-dimethylformamide (6 drops) in toluene (500 mL) was stirred for 16h at 25°C and concentrated in vacuo to afford 3-methoxyphenylacetyl chloride. The acid chloride was dissolved in chloroform (100 mL) and added to a solution of aminoacetaldehyde dimethyl acetal (32.1 g, 0.306 mol) and triethylamine (32.4 g, 0.320 mol in chloroform (500 mL) stirred at 5°C. The mixture was stirred at 25°C for 16h, washed with water, 1.5N hydrochloric acid and water, dried over magnesium sulfate and concentrated in vacuo to afford N-(2,2-dimethoxyethyl)-3-methoxybenzeneacetamide.

A solution of the benzeneacetamide (70 g, 0.277 mol) in acetic acid (180 mL) was added with stirring to concentrated hydrochloric acid (120 mL). The mixture was stirred for 16h, diluted with ice/water and filtered. The filter cake was dissolved in methylene chloride which was washed with water, dried with magnesium sulfate and concentrated in vacuo to afford 2,3-dihydro-8-methoxy-2-oxo-1H-3-benzazepine.

A mixture of 2,3-dihydro-8-methoxy-2-oxo-1H-3-benzazepine (12 g, 0.063 mol) and 10% palladium-on-carbon (1.2 g) in acetic acid (200 mL) was shaken in an atmosphere of hydrogen (413, 700 Pa), degassed, filtered and concentrated in vacuo. The residue was dissolved in methylene chloride, washed with water, dried with magnesium sulfate and concentrated in vacuo. The residue was triturated with ether and filtered to afford 8-methoxy-2-oxo-1H-3-benzazepine.

A suspension of 8-methoxy-2-oxo-1H-3-benzazepine (20.4 g, 0.105 M) in tetrahydrofuran (500 mL) was added to 1M borane in tetrahydrofuran (300 mL) stirred at 5°C. The mixture was heated to reflux for 2h, cooled, treated with 3N hydrochloric acid (300 ML), concentrated in vacuo to remove tetrahydrofuran and heated to reflux for lh. The mixture was concentrated in vacuo filtered and the filter cake was dissolved in methanol, heated to reflux, dried with magnesium sulfate and concentrated in vacuo to afford 7-methoxy-2,3,4,5-tetrahydro-1H-3-benzazepine hydrochloride, m.p. 229-231°C.

A mixture of 7-methoxy-2,3,4,5-tetrahydro-1H-3-benzazepine hydrochloride (2.1 g, 0.01 mol) and 48% hydrobromic acid (40 mL) was heated to reflux for 2.5h, concentrated in vacuo, and the residue was triturated with ether and recrystallized from methanol-ether to afford 7-hydroxy-2,3,4,5-tetrahydro-1H-3-benzazepine hydrobromide, m.p. 247-249°C.

## EXAMPLE 2

### 7-Bromo-8-hydroxy-3-methyl-2,3,4,5-tetrahydro-1H-3-benzazepine

A mixture of 7-methoxy-2,3,4,5-tetrahydro-1H-3-benzazepine hydrochloride (2.1 g, 0.01 mol), 37% formalin (3 mL) and 10% palladium-on-carbon (0.2 g) in methanol (50 mL) was shaken in an atmosphere of hydrogen (413, 700 Pa) for 4h. The mixture was degassed, filtered and concentrated in vacuo. The residue was crystallized from ethanol-ether to afford 7-methoxy-3-methyl-2,3,4,5-tetrahydro-1H-3-benzazepine hydrochloride, m.p. 193-194°C.

A solution of bromine (6.4 g, 0.04 mol) in acetic acid (40 mL) was added to a stirred solution of 7-methoxy-3-methyl-2,3,4,5-tetrahydro-1H-3-benzazepine hydrochloride (8.5 g, 0.037 mol) in acetic acid (70 mL). The mixture was warmed to 70°C for 1.5h, concentrated in vacuo and partitioned between aqueous sodium hydroxide and ethyl acetate. The ethyl acetate phase was washed, dried with magnesium sulfate and concentrated in vacuo The residue was converted to the hydrochloride and crystallized from ethanol to afford 7-bromo-8-methoxy-3-methyl-2,3,4,5-tetrahydro-1H-3-benzazepine hydrochloride, m.p. 231-233°C.

A solution of boron tribromide (6.5 g, 0.026 mol) in methylene chloride (16 mL) was added to a solution of 3-methyl-7-bromo-8-methoxy-2,3,4,5-tetrahydro-1H-3-benzazepine hydrochloride (3.5 g, 0.013 mol) in methylene chloride (100 mL) stirred at 5°C. The mixture was stirred at 25°C for 2h, treated with methanol and concentrated in vacuo. The residue was crystallized from methanolether to afford 7-bromo-8-hydroxy-3-methyl-2,3,4,5-tetrahydro-1H-3-benzazepine hydrobromide, m.p. 236°C.

## EXAMPLE 3

### 8-Hydroxy-3-methyl-7-phenylthio-2,3,4,5-tetrahydro-1H-3-benzazepine

7-Bromo-8-methoxy-3-methyl-2,3,4,5-tetrahydro-1H-3-benzazepine (7 g, 0.026 mol) was dissolved in ether (70 ml) and added slowly to a solution of n-butyllithium (0.096 m) in ether (50 mL) stirred at -75°C. The mixture was stirred for 45 minutes, treated with a solution of diphenyl disulfide (21 g, 0.096 mol) dissolved in ether (200 mL), and stirred for one hour at -75°C. The mixture was poured into a mixture of ice and concentrated hydrochloric acid and extracted with ether. The acidic, aqueous phase was basified with 40% aqueous sodium hydroxide and extracted with ether. The ether extract was washed, dried with magnesium sulfate and concentrated in vacuo. The residue was dissolved in absolute ethanol and converted to the hydrochloride to give 8-methoxy-3-methyl-7-phenylthio-2,3,4,5-tetrahydro-1H-3-benzazepine hydrochloride.

Following the general procedure of Example 2, 8-methoxy-3-methyl-7-phenylthio-2,3,4,5-tetrahydro-1H-3-benzazepine hydrochloride (3.5 g, 0.01 mol) was reacted with boron tribromide (7.5 g, 0.03 mol) and then with maleic acid to afford 8-methoxy-3-methyl-7-phenylthio-2,3,4,5-tetrahydro-1H-3-benzazepine maleate, m.p. 174-176°C.

EXAMPLE 4

7-Hydroxy-3-methyl-6-phenylthio-2,3,4,5-tetrahydro-1H-3-benzazepine

N-Butyllithium (1 M) in hexane (450 mL) was added to a solution of N,N-dimethyl-3-methoxybenzylamine (100 g, 0.6 mol) in tetrahydrofuran (1 L) stirred at 0°C. The mixture was cooled to -78°C, treated with a solution of diphenyl disulfide (218 g, 1 mol) in ether (1 L), allowed to warm to room temperature and stirred for 4h. The mixture was concentrated in vacuo and partitioned between 10% hydrochloric acid and ether. The acidic, aqueous phase was basified with 40% aqueous sodium hydroxide and extracted with methylene chloride. The methylene chloride phase was washed, dried with sodium sulfate and concentrated in vacuo. The residue was chromatographed on silica gel eluted with methanol/chloroform (0-20%) to afford N,N-dimethyl-2-phenylthio-3-methoxy-benzylamine.

N,N-Dimethyl-2-phenylthio-3-methoxy-benzylamine was converted to 2-phenylthio-3-methoxyphenylacetonitrate by the general method of D.S. Kasdan, J.A. Schwartz and H. Rapoport, J. Org. Chem., 47 2638 (1982)

2-Phenylthio-3-methoxy-phenylacetonitrite (9.2 g, 36 mmol) was dissolved in ethanol (75 mL), treated with 10% aqueous sodium hydroxide (108 mL) and heated to 95°C for 18h. The mixture was concentrated in vacuo acidified with 10% hydrochloric acid and extracted with ethyl acetate. The ethylacetate phase was dried with sodium sulfate and concentrated in vacuo to give 2-phenylthio-3-methoxyphenylacetic acid.

Following the general procedure of Example 1, 3-methoxy-2-phenylthiophenylacetic acid was converted to 7-methoxy-6-phenylthio-2,3,4,5-tetrahydro-1H-3-benzazepine.

7-Methoxy-6-phenylthio-2,3,4,5-tetrahydro-1H-3-benzazepine (2 g, 7 mmol) was dissolved in 98% formic acid (50 mL), treated with 37% formaldehyde (10 mL) and heated to 95°C for 5h.

The mixture was poured into ice water, basified with 10% aqueous sodium hydroxide and extracted with ethyl acetate. The ethyl acetate phase was washed, dried with sodium sulfate and concentrated in vacuo. The residue was chromatographed on silica gel eluted with methanolchloroform (0-10%) to give the desired product which was treated with hydrogen chloride to give 7-methoxy-3-methyl-6-phenylthio-2,3,4,5-tetrahydro-1H-3-benzazepine hydrochloride, m.p. 211-212°C.

7-Methoxy-3-methyl 6-phenylthio-2,3,4,5-tetrahydro-1H-3-benzazepine (1.5 g, 5 mmol) was dissolved in methanesulfonic acid (100 mL), treated with methionine (4 g) and stirred at 25°C for 3 days. The mixture was poured into ice water, adjusted to pH 8 and extracted with ethyl acetate. The ethyl acetate phase was washed, dried with sodium sulfate and concentrated in vacuo. The residue was chromatographed on silica eluted wth methanolchloroform (2-30%) to give the desired product which was treated with hydrogen chloride to give 7-hydroxy-3-methyl-6-phenylthio-2,3,4,5-tetrahydro-1H-3-benzazepine hydrochloride: m.p. 153-155°C.

EXAMPLE 5

8-Hydroxy-7-methylthio-2,3,4,5-tetrahydro-1H-3-benzazepine

A mixture of 7-methoxy-2,3,4,5-tetrahydro-1H-3-benzazepine hydrochloride (4.3 g, 0.02 mol) and sodium acetate (3.3 g, 0.04 mol) in acetic anhydride (13 mL) was refluxed for 16h, concentrated in vacuo and partitioned between methylene chloride and water. The organic phase was dried over magnesium sulfate, filtered and concentrated in vacuo to give 3-acetyl-7-methoxy-2,3,4,5-tetrahydro-1H-3-benzazepine, m.p. 89-90°C.

3-Acetyl-7-methoxy-2,3,4,5-tetrahydro-1H-3-benzazepine (2.3 g, 0.01 mol) was added to chlorosulfonic acid (6 mL) stirred at 0°C and the mixture was allowed to warm to 25°C and stirred for 16h. The reaction was poured into ice water and extracted with methylene chloride. The combined methylene chloride extracts were combined, washed, dried over magnesium sulfate and concentrated in vacuo to give 3-acetyl-7-chlorosulfonyl-8-methoxy-2,3,4,5-tetrahydro-1H-3-benzazepine, m.p. 153-160°C.

3-Acetyl-7-chlorosulfonyl-8-methoxy-2,3,4,5-tetrahydro-1H-3-benzazepine (4 g, 0.013 M) was dissolved in glacial acetic acid (80 mL), treated with stannous chloride dihydrate (11.6 g., 0.05 mol) and concentrated hydrochloric acid (16 mL) and stirred at 75°C for 1h. The mixture was cooled, poured into ice water and extracted with ethyl acetate. The combined ethyl acetate extract was washed, dried with magnesium sulfate and concentrated in vacuo to afford a mixture of 3-acetyl-7-mercapto-8-methoxy-2,3,4,5-tetrahydro-1H-3-benzazepine and the corresponding disulfide.

The crude mixture (3 g) was dissolved in ethanol and treated with sodium borohydride (2 g, 0.05 mol)

to effect reduction of the disulfide to the mercaptan. Methyl iodide (2 g, 0.01 mol) was added and the reaction stirred at 25°C for 1h. The mixture was concentrated, partitioned between water and methylene chloride and the combined methylene chloride extract was washed, dried over magnesium sulfate and concentrated in vacuo to give 3-acetyl-7-methylthio-8-methoxy-2,3,4,5-tetrahydro-1H-3-benzazepine, m.p. 138-140°C.

A mixture of 3-acetyl-7-methylthio-8-methoxy-2,3,4,5-tetrahydro-1H-3-benzazepine and 3N hydrochloric acid is heated to reflux for 16h and concentrated in vacuo to afford 7-methylthio-8-methoxy-2,3,4,5-tetrahydro-1H-3-benzazepine hydrochloride.

Following the general procedure of Example 7, 7-methylthio-8-methoxy-2,3,4,5-tetrahydro-1H-3-benzazepine hydrochloride is treated with methionine in methanesulfonic acid to afford 7-methylthio-8-methoxy-2,3,4,5-tetrahydro-1H-3-benzazepine hydrochloride.

EXAMPLE 6

3-Methyl-7-phenylthio-2,3,4,5-tetrahydro-1H-3-benzazepine

Following the general procedure of Example 3, 7-bromo-3-methyl-2,3,4,5-tetrahydro-1H-3-benzazepine was treated with n-butyllithium and diphenyl disulfide to give 3-methyl-7-phenylthio-2,3,4,5-tetrahydro-1H-3-benzazepine fumarate, m.p. 177-178°C.

EXAMPLE 7

8-Hydroxy-7-trifluoromethylthio-2,3,4,5-tetrahydro-1H-3-benzazepine

Following the general procedure of Example 3, 7-bromo-8-methoxy-2,3,4,5-tetrahydro-1H-3-benzazepine is treated with butyllithium and then with trifluoromethylsulfenyl chloride to give 8-methoxy-7-trifluoromethylthio-2,3,4,5-tetrahydro-1H-3-benzazepine.

8-Methoxy-7-trifluoromethylthio-2,3,4,5-tetrahydro-1H-3-benzazepine and methionine are dissolved in methanesulfonic acid and stirred. The mixture is diluted, made alkaline with 5% aqueous sodium carbonate and extracted with methylene chloride to give 8-hydroxy-7-trifluoromethylthio-2,3,4,5-tetrahydro-1H-3-benzazepine.

EXAMPLE 8

8-Hydroxy-3-methyl-7-phenylsulfinyl-2,3,4,5-tetrahydro-1H-3-benzazepine

Following the general procedure of Example 7, 7-bromo-8-methoxy-2,3,4,5-tetrahydro-1H-3-benzazepine is treated with butyllithium and diphenyl disulfide to give 8-methoxy-7-phenylthio-2,3,4,5-tetrahydro-1H-3-benzazepine. A mixture of 8-methoxy-7-phenylthio-2,3,4,5-tetrahydro-1H-3-benzazepine and trifluoracetic anhydride in methylene chloride is stirred for 1 hour, treated with methanol and concentrated in vacuo to give 8-methoxy-7-phenylthio-3-trifluoroacetyl-2,3,4,5-tetrahydro-1H-3-benzazepine.

8-Methoxy-7-phenylthio-3-trifluoroacetyl-2,3,4,5-tetrahydro-1H-3-benzazepine is treated with one equivalent of 3-chloroperoxybenzoic acid in chloroform and stirred.

The mixture is washed with 5% aqueous sodium carbonate and water, dried with sodium sulfate and concentrated in vacuo to give 8-methoxy-7-phenylsulfinyl-3-trifluoroacetyl-2,3,4,5-tetrahydro-1H-3-benzazepine. The trifluoroacetamide is dissolved in methanol and treated with aqueous sodium hydroxide to give 8-methoxy-7-phenylsulfinyl-2,3,4,5-tetrahydro-1H-3-benzazepine.

Following the general procedure of Example 2, 8-methoxy-7-phenylsulfinyl-2,3,4,5-tetrahydro-1H-3-benzazepine is methylated to give 8-methoxy-3-methyl-7-phenylsufinyl-2,3,4,5-tetrahydro-1H-3-benzazepine. Following the general procedure of Example 4, 8-methoxy-3-methyl-7-phenylsulfinyl-2,3,4,5-tetrahydro-1H-3-benzazepine is treated with methionine and methanesulfonic acid to give 8-hydroxy-3-methyl-7-phenylsulfinyl-2,3,4,5-tetrahydro-1H-3-benzazepine.

EXAMPLE 9

8-Hydroxy-3-methyl-7-phenylsulfonyl-2,3,4,5-tetrahydro-1H-3-benzazepine

Following the general procedure of Example 8, 8-methoxy-7-phenylthio-2,3,4,5-tetrahydro-1H-3-benzazepine is treated with two equivalents of 3-chlorperoxybenzoic acid to give 8-methoxy-7-phenylsulfonyl-2,3,4,5-tetrahydro-1H-3-benzazepine.

Following the general procedure of Example 8, 8-methoxy-7-phenylsulfonyl-2,3,4,5-tetrahydro-1H-3-benzazepine is methylated and treated with methionine to give 8-hydroxy-3-methyl-7-phenylsulfonyl-2,3,4,5-tetrahydro-1H-3-benzazepine.

EXAMPLE 10

8-Hydroxy-7-4-(hydroxyphenyl)sulfonyl-2,3,4,5-tetrahydro-1H-3-benzazepine

Following the general procedure of Examples 7 and 9, 7-bromo-8-methoxy-2,3,4,5-tetrahydro-1H-3-benzazepine is treated with butyllithium and then with di(4-methoxyphenyl) disulfide to give 8-methoxy-7-(4-methoxyphenyl) sulfonyl-2,3,4,5-tetrahydro-1H-3-benzazepine.

Following the general procedure of Example 1, 8-methoxy-7-(4-methoxyphenyl)sulfonyl-2,3,4,5-tetrahydro-1H-3-benzazepine is treated with hydrobromic acid to give 8-hydroxy-7-(4-hydroxyphenyl)-sulfonyl-2,3,4,5-tetrahydro-1H-3-benzazepine.

EXAMPLE 11

3-Methyl-8-nitro-7-phenylthio-2,3,4,5-tetrahydro-1H-3-benzazepine

7-Chloro-3-methyl-8-nitro-2,3,4,5-tetrahydro-1H-3-benzazepine hydrochloride (3 g, 0.008 mol) was converted to the free base and added to a mixture of sodium hydride (0.6 g), thiophenol (1.3 g) and dimethylformamide (21 ml) stirred at 25°C. The mixture was stirred for one hour, poured into ice-water, neutralized and extracted with ether. The ether extract was washed, dried with sodium sulfate and concentrated in vacuo. The residue was treated with hydrogen chloride and the resulting salt was triturated with isopropanol and recrystallized from ethanol to give 3-methyl-8-nitro-7-phenylthio-2,3,4,5-tetrahydro-1H-3-benzazepine hydrochloride, m.p. 233-234°C.

EXAMPLE 12

3-Methyl-7-phenylthio-2,3,4,5-tetrahydro-1H-3-benzazepine fumarate (10 mg) is mixed with 75 mg of lactose and 2 mg of magnesium stearate. The resulting mixture is filled into a hard gelatin capsule.

## Claims

1. The use of a compound of the formula (I):

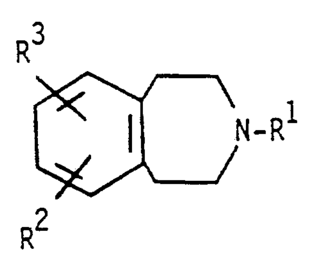

(I)

in which:

$R^1$ is hydrogen, $C_1$-$C_5$ alkyl or $C_3$-$C_5$ alkenyl;

$R^2$ is hydrogen, halogen, $CF_3$, $C_1$-$C_6$ alkyl or $R^4O$;

$R^3$ is halogen, $CF_3$, $C_1$-$C_6$ alkyl, hydroxy, $C_1$-$C_6$ alkoxy, S-($C_1$-$C_6$) alkyl, S-trifluoromethyl, $NO_2$, $SO_n$-

phenyl where the phenyl is optionally substituted by hydroxy, $C_1$-$C_6$ alkoxy, halogen, $CF_3$, $C_1$-$C_6$ alkyl, $SO_n$($C_1$-$C_6$) alkyl, $SO_nCF_3$, $SO_n$-phenyl or $SO_2NR^5R^6$;

$R^4$ is hydrogen, $C_1$-$C_6$ alkyl, or $C_1$-$C_6$ alkanoyl;

$R^5$ and $R^6$ are hydrogen or $C_1$-$C_6$ alkyl; and

n is 0, 1 or 2;

provided $R^2$ is not hydrogen when $R^3$ is S-phenyl in the 6-position and provided that when $R^2$ is hydroxy in the 8-position and $R^1$ is $C_1$-$C_6$ alkyl, $R^3$ may be halogen or $CF_3$ only if it is in the 6-or 9-position; or a pharmaceutically acceptable addition salt thereof, in the manufacture of a medicament for the treatment of gastrointestinal motility disorders.

2. The use of a compound of the formula (I) as defined in claim 1 in which $R^2$ is hydrogen, hydroxy or methoxy; $R^3$ is $SO_n$-phenyl or S-($C_1$-$C_6$) alkyl, and $R^1$ is hydrogen or methyl.

3. The use of a compound of the formula (I) as defined in claim 1 in which $R^3$ is in the 7-position.

4. The use of a compound of the formula (I) as defined in claim 1 in which $R^3$ is in the 6-position.

5. The use of a compound of the formula (I) as defined in claim 1 in which $R^3$ is in the 7-position and $R^2$ is in the 8-position.

6. The use of a compound of the formula (I) as defined in claim 1 in which $R^3$ is in the 6-position and $R^2$ is in the 7-position.

7. The use of a compound of the formula (I) as defined in claim 1 which is 3-methyl-7-phenylthio-2,3,4,5-tetrahydro-1H-3-benzazepine.

8. The use of a compound of the formula (I) as defined in claim 1 which is 8-hydroxy-3-methyl-7-phenylthio-2,3,4,5-tetrahydro-1H-3-benzazepine.

9. The use of a compound of the formula (I) as defined in claim 1 which is 7-hydroxy-3-methyl-6-phenylthio-2,3,4,5-tetrahydro-1H-3-benzazepine.

10. A pharmaceutical composition comprising a pharmaceutically acceptable carrier and a compound of formula (II):

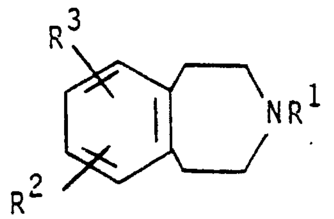

(II)

in which:

$R^1$ is hydrogen, $C_1$-$C_6$ alkyl or $C_3$-$C_5$ alkenyl;

$R^2$ is hydrogen, halogen, $CF_3$, $C_1$-$C_6$ alkyl or $R^4O$;

$R^3$ is halogen, $CF_3$, $NO_2$, S-($C_1$-$C_6$) alkyl, S-trifluoromethyl, $SO_n$-phenyl where the phenyl is optionally substituted by hydroxy, $C_1$-$C_6$ alkoxy, halogen, $CF_3$, $C_1$-$C_6$ alkyl, $SO_n$($C_1$-$C_6$) alkyl, $SO_nCF_3$, $SO_n$phenyl or $SO_2NR^5R^6$;

$R^4$ is hydrogen, $C_1$-$C_6$ alkyl, or $C_1$-$C_6$alkanoyl;

$R^5$ and $R^6$ are hydrogen or $C_1$-$C_6$ alkyl; and

n is 1 or 2, or if $R^3$ is in the 7-or 8-position, n may be 0;

provided that when $R^2$ is hydroxy in the 8-position and $R^1$ is $C_1$-$C_6$ alkyl, $R^3$ may be halogen or $CF_3$ only if it is in the 6-or 9-position; or a pharmaceutically acceptable acid addition salt thereof.

11. A pharmaceutical composition according to claim 10 for use in the treatment of gastrointestinal motility disorders.

12. A pharmaceutical composition according to claim 10 or 11 in which the compound of formula (II) is 3-methyl-7-phenylthio-2,3,4,5-tetrahydro-1H-3-benzazepine.

13. A pharmaceutical composition according to claim 10 or 11 in which the compound of formula (II) is 8-hydroxy-3-methyl-7-phenylthio-2,3,4,5-tetrahydro-1H-3-benzazepine.

Claims for the following Contracting States : GR ; ES

1. The use of a compound of the formula (I):

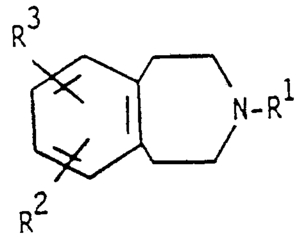

(I)

in which:

$R^1$ is hydrogen, $C_1$-$C_6$ alkyl or $C_3$-$C_5$ alkenyl;

$R^2$ is hydrogen, halogen, $CF_3$, $C_1$-$C_6$ alkyl or $R^4O$;

$R^3$ is halogen, $CF_3$, $C_1$-$C_6$ alkyl, hydroxy, $C_1$-$C_6$ alkoxy, S-($C_1$-$C_5$) alkyl, S-trifluoromethyl, $NO_2$, $SO_n$-phenyl where the phenyl is optionally substituted by hydroxy, $C_1$-$C_6$ alkoxy, halogen, $CF_3$, $C_1$-$C_6$ alkyl, $SO_n$($C_1$-$C_6$) alkyl, $SO_nCF_3$ $SO_n$-phenyl or $SO_2NR^5R^6$;

$R^4$ is hydrogen, $C_1$-$C_6$ alkyl, or $C_1$-$C_6$ alkanoyl;

$R^5$ and $R^6$ are hydrogen or $C_1$-$C_6$ alkyl; and

n is 0, 1 or 2;

provided $R^2$ is not hydrogen when $R^3$ is S-phenyl in the 6-position and provided that when $R^2$ is hydroxy in the 8-position and $R^1$ is $C_1$-$C_6$ alkyl, $R^3$ may be halogen or $CF_3$ only if it is in the 6-or 9-position; or a pharmaceutically acceptable addition salt thereof, in the manufacture of a medicament for the treatment of gastrointestinal motility disorders.

2. The use of a compound of the formula (I) as defined in claim 1 in which $R^2$ is hydrogen, hydroxy or methoxy; $R^3$ is $SO_n$-phenyl or S-($C_1$-$C_6$) alkyl, and $R^1$ is hydrogen or methyl.

3. The use of a compound of the formula (1) as defined in claim 1 in which $R^3$ is in the 7-position.

4. The use of a compound of the formula (I) as defined in claim 1 in which $R^3$ is in the 6-position.

5. The use of a compound of the formula (I) as defined in claim 1 in which $R^3$ is in the 7-position and $R^2$ is in the 8-position.

6. The use of a compound of the formula (I) as defined in claim 1 in which $R^3$ is in the 6-position and $R^2$ is in the 7-position.

7. The use of a compound of the formula (I) as defined in claim 1 which is 3-methyl-7-phenylthio-2,3,4,5-tetrahydro-1H-3-benzazepine.

8. The use of a compound of the formula (I) as defined in claim 1 which is 8-hydroxy-3-methyl-7-phenylthio-2,3,4,5-tetrahydro-1H-3-benzazepine.

9. The use of a compound of the formula (I) as defined in claim 1 which is 7-hydroxy-3-methyl-6-phenylthio-2,3,4,5-tetrahydro-1H-3-benzazepine.

10. A process for preparing a pharmaceutical composition which comprises bringing into association a compound of the formula (II):

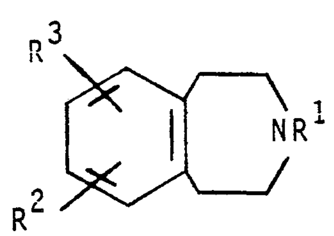

(II)

in which:

$R^1$ is hydrogen, $C_1$-$C_6$ alkyl or $C_3$-$C_5$ alkenyl;

$R^2$ is hydrogen, halogen, $CF_3$, $C_1$-$C_6$ alkyl or $R^4O$;

$R^3$ is halogen, $CF_3$, $NO_2$, S-($C_1$-$C_6$) alkyl, S-trifluoromethyl, $SO_n$-phenyl where the phenyl is optionally substituted by hydroxy, $C_1$-$C_6$ alkoxy, halogen, $CF_3$, $C_1$-$C_6$ alkyl, $SO_n$($C_1$-$C_6$) alkyl, $SO_nCF_3$, $SO_n$phenyl or

$SO_2NR^5R^6$;

$R^4$ is hydrogen, $C_1$-$C_6$ alkyl, or $C_1$-$C_6$ alkanoyl;

$R^5$ and $R^6$ are hydrogen or $C_1$-$C_6$ alkyl; and

n is 1 or 2, or if $R^3$ is in the 7-or 8-position, n may be 0;

provided that when $R^2$ is hydroxy in the 8-position and $R^1$ is $C_1$-$C_6$ alkyl, $R^3$ may be halogen or $CF_3$ only if it is in the 6-or 9-position; or a pharmaceutically acceptable acid addition salt thereof; and a pharmaceutically acceptable carrier.

11. A process for preparing a pharmaceutical composition for treatment of gastrointestinal motility disorders which comprises bringing into association a compound of the formula (II) as defined in claim 10 or a pharmaceutically acceptable acid addition salt thereof, and a pharmaceutically acceptable carrier.

12. A process according to claim 10 or 11 in which the compound of formula (II) is 3-methyl-7-phenylthio-2,3,4,5-tetrahydro-1H-3-benzazepine.

13. A process according to claim 10 or 11 in which the compound of formula (II) is 8-hydroxy-3-methyl-7-phenylthio-2,3,4,5-tetrahydro-1H-3-benzazepine.